# EUROPEAN PATENT APPLICATION

(11) **EP 4 573 920 A1**
(43) Date of publication of application: **25.06.2025**
(21) Application number: 23307361.8
(22) Date of filing: 22.12.2023
(51) Int. Cl.: A23D 7/01, A23D 9/013, A61K 8/37, A61K 47/12, A61K 47/14

(54) **TEXTURIZING COMPLEX COMPRISING OLEOYLETHANOLAMIDE AND AT LEAST ONE FATTY SUBSTANCE**

(71) Applicant: Centre National de la Recherche Scientifique, 75016 Paris (FR); Université de Strasbourg, 67081 Strasbourg Cedex (FR)
(72) Inventor: Mésini, Philippe, 67230 Sand (FR); Schwaller, Duncan, 59110 La Madeleine (FR); Yilmazer, Senem, 67201 Eckbolsheim (FR)
(74) Representative: Novagraaf Technologies

(57) **Abstract**

The present invention belongs to the field of texturizing agents and more particularly their applications in several fields like pharmaceutical, food, cosmetic or lube application.
The prevent invention relates to a texturizing complex comprising oleoylethanolamide and at least one fatty substance and its process of manufacture. The invention also relates to a composition, such as pharmaceutical, food, cosmetic or lube composition.

## Description

### Technical field

The present invention belongs to the field of texturizing agents and more particularly their applications in several fields like pharmaceutical, food, cosmetic or lube application.

The present invention relates to a texturizing complex comprising oleoylethanolamide and at least one fatty substance and its process of manufacture.

The present invention also relates to a composition, such as pharmaceutical, food, cosmetic or lube composition.

In the description below, references between **[ ]** refer to the list of references at the end of the examples.

### Technical background

Texturizing agents are present in a wide range of compositions, so their use covers many fields.

For example, in many food applications, the lipid phase needs to be structured, i.e. given desirable visco-elastic properties. This role is assigned to the solid fraction of fats, which is made up of saturated fatty acids and, to a lesser extent, trans-unsaturated fatty acids, which have negative effects on health [1-4]. There is therefore pressure, both from public health authorities and from consumers, to replace these solid fats with healthier substitutes, while retaining the texture of the lipid phase [7-11].

In cosmetics, natural oils or at least bio-based solvents are increasingly used in formulations. There is also a need to find new texturizing agents for these oils that are also biosourced, harmless, and environment friendly.

### Detailed description of the invention

The inventors have developed a new texturizing complex using oleoylethanolamide of formula (I):

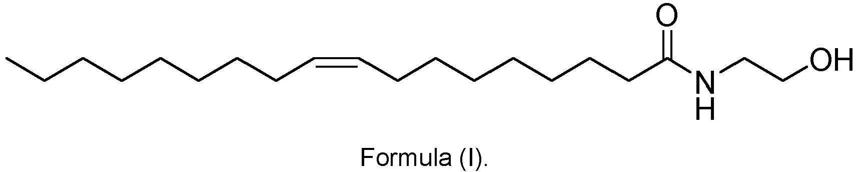

Oleoylethanolamide is a natural, endogenous product, i.e. present in our bodies. This product has been FDA-approved since 2017 and sold as a dietary supplement for its physiological effect of inhibiting the sensation of hunger and thus naturally limiting food intake and weight gain [12-16].

The inventors surprisingly found out that oleoylethanolamide in combination with at least one fatty substance enables the production of a texturizing complex to give more viscosity or firmness to a composition. The inventors have thus made an alternative solution to already known texturizing agents without the associated drawbacks mentioned above.

According to the description "texturizing agent" means any additive which increases the consistency and the firmness of a composition. For example a texturizing agent may be made of starch, natural gum (guar gum, arabic gum, tragacanth gum), casein, phytocolloid (carragenates, alginates, agar), carboxyvinyl polymers (carbomer), acrylic copolymer, polyacrylamide, polysaccharide, dextrin palmitate, clays such as modified clays (bentones) and hydrophobic silica, cellulose oleogelators (ethylcellulose [17], phytosterols/sterol esters mixtures [18], monoglycerides [19], waxes [20], fatty alcohols/fatty acids [21], 12-hydroxystearic acid [22]).

The invention thus relates in a first aspect to a texturizing complex comprising oleoylethanolamide and at least one fatty substance.

According to the invention, "texturizing complex" means a combination of oleoylethanolamide, at least one fatty substance and optionally one wax and/or an excipient. The texturizing complex may advantageously replace or reinforce the effect of a texturizing agent in a composition, such as pharmaceutical, food, cosmetic or lube composition.

Advantageously, the texturizing complex may be in gel form.

According to the description, "gel form" means the complex has a viscoelastic behaviour.

Advantageously, the mass concentration of the at least one fatty substance relative to the total mass of the texturizing complex may be in the range from 70 to 99.9 %, preferably from 80 to 99 %, and more preferably from 90 to 99 %.

Advantageously, the mass concentration of oleoylethanolamide relative to the total mass of the texturizing complex may be in the range from 0.1 to 30 %, preferably from 1 to 20%, and more preferably from 1 to 10 %.

According to the description, "fatty substance" means any substance composed mainly of glycerides or other lipids, insoluble in water and soluble in non-polar organic solvents. The fatty substance may be natural or synthetic, preferably the fatty substance may be mineral, vegetable or animal origin.

Advantageously, the fatty substance may be selected from oil, butter, fat, emulsion and mixtures thereof.

According to the description, "oil" means any fatty flammable substance of plant, animal or mineral origin, immiscible in water, miscible in non-polar organic solvent, generally liquid above 10°C, freezing or solidifying at lower temperatures.

According to the description, "miscible" means that the resulting mixture is homogeneous, i.e., a single phase is observed visually and "immiscible" means that the resulting mixture is heterogeneous, ie, at least two phases can be observed visually.

Advantageously, the oil may be selected from natural oil, synthetic oil and mixtures thereof, preferably from vegetable oil, animal oil, mineral oil and mixtures thereof.

Advantageously, the oil may be selected from vegetable oil, preferably from rapeseed/canola, sunflower, camellia, olive, coconut, argan, jojoba, castor, soybean, corn, safflower, flaxseed, almond, peanut, algal, palm, palm stearin, palm kernel oil, avocado, apricot, wheat germ, cottonseed, vegetable squalane, caprylic/capric triglycerides, echium, rice bran, sesame, grapeseed, helianthus annuus seed oil, green tea seed oil, macadamia nut oil, rose hips oil, hydrogenated oil, sea buckthorn oil, argan tree kernel oil, lavender oil and mixtures thereof.

Advantageously, the oil may be selected from animal oil, preferably from fish oil.

Advantageously, the oil may be selected from natural or synthetic ester-based oil, preferably from isopropyl myristate, ethyl laurate, ethyl myristate, isopropyl palmitate, butyl laurate, hexyl laurate, caprylic/capric triglyceride, butyl glycol dicaprylate/dicaprate, isopropyl myristate, triethylhexanoin, cetyl ethylhexanoate, octyldodecanol, polyol ester and mixtures thereof.

Advantageously, the oil may be selected from mineral oil or synthetic oil, preferably from hydrocarbon-based oils including cyclododecane, isooctane, liquid paraffin, Vaseline, hydrogenated polydecene, cyclooctane, cyclopentane, and squalene, isododecane, C₁₀ to C₄₀ alkanes mixtures, squalane and mixtures thereof.

According to the description, "butter" means any dairy product made from churned cream or any solid fat from vegetal origin.

Advantageously, the butter may be selected from clarified butter, ghee, shea butter, cocoa butter, peanut butter, avocado butter, nut butter and mixtures thereof, preferably the butter may be shea butter.

According to the description, "fat" means any solid or semi-solid substance, as distinguished from an oil, generally highly flammable, insoluble in water but soluble in non-polar organic solvent (for example in diethyl ether), viscous to the touch, of animal or vegetable origin, and which melts between 20°C and 50°C.

Advantageously, the fat may be selected from duck fat, ox tallow, sheep tallow, lard and mixtures thereof.

According to the description, "emulsion" means any more or less stable dispersion, in the form of microscopic droplets, of one liquid in a second, immiscible liquid.

Advantageously, the emulsion may be selected from water in oil emulsion such as margarine.

Advantageously, the texturizing agent may further comprise at least one wax.

According to the description, "wax" means any of numerous substances of plant or animal origin that differ from fats in being less greasy, harder, and crumblier (such as fatty acids, alcohols, esters and saturated hydrocarbons). For example, waxes may be naturally occurring waxes, synthetic waxes or mixtures thereof.

Preferably the wax may be selected from candelilla wax, carnauba wax/Brazil wax, bee wax, rice bran oil wax, sugarcane wax, lignite wax, shellac wax, red tail fat, ceresin, petroleum jelly, hydrogenated Jojoba wax and mixtures thereof. More preferably, the max may be selected from candelilla wax, carnauba wax/Brazil wax, bee wax, lanolin, bayberry, sugarcane, microcrystalline, petrolatum and carbowax and mixtures thereof.

Advantageously, the mass concentration of the at least one wax relative to the total mass of the texturizing complex is in the range from 0.01 to 20 %, preferably from 0.1 to 10 %, more preferably 0.1 to 1 %.

Advantageously, the texturizing complex further comprises at least one excipient, preferably a food, cosmetic, pharmaceutically acceptable or lube excipient.

According to the description, "excipient" means any component of a composition which does not confer its therapeutic or preventive properties, but which may play a role in the absorption (assimilation) and stability of an active substance, as well as in its appearance, colour and taste.

Advantageously, the texturizing complex according to the invention may comprise an elastic modulus comprised from 1.10³ to 1.10⁷ Pa, preferably 1.10³ to 1.10⁶ Pa, more preferably 1.10³ to 1.10⁵ Pa. The incorporation of at least one wax in the texturizing complex according to the invention may have a positive impact on the value of the elastic modulus and/or the stability.

The elastic modulus may be measured according to the following method. The real and imaginary parts of the complex shear modulus were measured with a commercial stress-controlled rheometer (Haake, Mars III), in the oscillatory mode and with a double Couette cell (DG41, Haake). The temperature was regulated by a heated bath (Haake F3) and controlled between 20 and 80 °C, within ± 0.05 °C. The experiments consisted in following, at a given frequency (1Hz), the evolution of the complex shear modulus of the sample when temperature was progressively decreased or increased between 80 to 20 °C at a rate of 0.25 °C/min. For all shear measurements the applied stress was between 0,05 and 0,1 Pa to ensure the data belong in the linear response regime. At room temperature, when the sample is gelled, a frequency sweep is then conducted from 10 Hz to 0,01 Hz. Then the heating phase takes place from 20 °C to 80 °C at a rate of 0,25 °C/min.

In a first variant, the texturizing complex according to the invention may comprise from 0.5 to 10 % of oleoylethanolamide, from 89.5 to 98.9 % of oil and from 0.1 to 0.5 % of wax.

In a second variant, the texturizing complex according to the invention may comprise from 0.5 to 10 % of oleoylethanolamide, from 89.5 to 98.9 % of butter and from 0.1 to 0.5 % of wax.

In a third variant, the texturizing complex according to the invention may comprise from 0.5 to 10 % of oleoylethanolamide, from 89.5 to 98.9 % of fat and from 0.1 to 0.5 % of wax.

The invention relates in a second aspect to a food composition comprising a texturizing complex according to the invention.

Advantageously, the food composition according to the invention may comprise from 3 to 99.9 % of texturizing complex relative to the total mass of the food composition.

Advantageously, the food excipient may be selected from a pigment, a dye, a natural or artificial colorant, an aroma, a filler, a texturizing agent, a preservative, an emulsifier, a sweetening agent, a flavouring agent, an anti-caking agent and mixtures thereof.

The invention relates in a third aspect to a cosmetic composition comprising a texturizing complex according to the invention.

Advantageously, the cosmetic composition according to the invention may comprise from 20 to 99.9 % of texturizing complex relative to the total mass of the cosmetic composition.

Advantageously, the cosmetic excipient may be selected from a pigment, a perfume, a filler, an emollient, a moisturizer, a stabilizing agent, a humectant, a film-forming agent, a texturizing agent, a cosmetic active ingredient, a UV filter, an antimicrobial agent, a preservative, an emulsifier, a fragrance, a whitening agent, and mixtures thereof.

The invention relates in a fourth aspect to a pharmaceutical composition comprising a texturizing complex according to the invention and a therapeutically active agent.

According to the description, "therapeutically active agent" means any agent tending to cure or prevent a disease or restore health.

Advantageously, the pharmaceutical composition according to the invention may comprise from 5 to 99 % of texturizing complex relative to the total mass of the pharmaceutical composition.

Advantageously, the pharmaceutically acceptable excipient may be selected from a filler, a UV filter (such as UVA and/or UVB filter), an antimicrobial agent, a preservative, an emulsifier, a binding agent, a disintegrant, a coating agent, a lubrication agent, and mixtures thereof.

Advantageously, the pharmaceutical composition according to the invention may be a medicament. The pharmaceutical composition according to the invention may be for use as a medicament.

The invention relates in another aspect to a process to manufacture a texturizing complex according to the invention wherein the process comprises:
a) a step of bringing into contact oleoylethanolamide and a fatty substance;
b) a step of gradually increasing the temperature of the mixture obtained in step a) until the dissolution of the oleoylethanolamide;
c) a step of homogenisation the mixture obtained in step b) to obtain a homogeneous mixture;
d) a step of gradually decreasing the temperature of the homogeneous mixture until the texturizing complex according to the invention is formed.

Advantageously, step b) and step c) may be implemented simultaneously or consecutively.

According to the description, "a step of homogenisation" means a step carried out by stirring, in the form of mechanical stirring or ultrasonic homogenizer stirring, by any mixer or homogenizer.

### EXAMPLE

### General information

The stability of the texturizing complex over time was assessed visually from the upside-down vial method, meaning the vial in which the texturizing complex was formed was put upside-down and 3 different visual aspects of aging were looked for. Firstly, no visual changes of the texturizing complex from the moment of its formation until the day of inspection, meaning the texturizing complex is stable at a given temperature during that period of time. Secondly, there is syneresis of the fatty substance, but the aspect of the texturizing complex itself is the same as the day of its formation, at a given temperature. And finally, the fatty substance is seeping from the texturizing complex leading to structural and visual deformation of the texturizing complex, which means the texturizing complex isn't stable in those conditions, at a given temperature, and is called demixing.

### Example 1: Synthesis of oleoylethanolamide (OEA)

The synthesis of oleoylethanolamide (OEA) was carried out according to the following scheme.

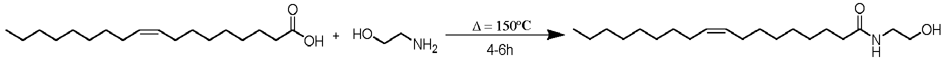

Oleic acid (1 equivalent) and ethanolamine (10 equivalents) were mixed in a round bottom flask and the mixture was stirred at 150°C overnight with a condenser as a safety precaution. After cooling, the reaction mixture was dissolved in ethyl acetate, then washed twice with a 1 M HCl solution, followed by a saturated NaCl solution. The organic phase was dried with anhydrous sodium sulfate, filtered and the solvent evaporated under reduced pressure. Finally, OEA was recrystallized in a minimum of acetone/water, then filtered and washed with cold water. After drying on a vacuum ramp, pure OEA was obtained in the form of white crystals in yields of between 30 and 50%.

### Example 2: OEA/Oil gel preparation

A typical preparation for OEA/Oil gels are carried out as followed. Briefly, OEA is weighted based on the desired concentration, then the desired oil is weighted in accordance with the quantity and concentration of the gel. The mixture is then heated at 70°C for 5 to 10 min until complete solubilisation of OEA in the oil. The resulting solution is then cooled to 20°C (or below for low OEA concentration gels) either slowly (simply left on the bench/fume hood) or rapidly (cooling with ice bath or stream of cold water).

### Example 3: OEA/sunflower oil composition

A mixture of OEA and sunflower oil, with an OEA concentration of 4% by weight, is heated for 10 min at 70°C, cooled to 20°C and left at this temperature for 1 h. The resulting composition is an opalescent white gel. This gel is stable for at least 3 months at 20°C and does not show any syneresis. The resulting composition has an elastic modulus of 10⁴ Pa.

### Example 4: OEA/rapeseed oil composition

A mixture of OEA and rapeseed oil, with an OEA concentration of 6% by weight, is heated for 10 min at 70°C, cooled to 20°C and then left at this temperature for 1 hour. The resulting composition is an opalescent white/yellow gel. This gel is stable for at least 3 months at 20°C and does not show any syneresis. The resulting composition has an elastic modulus of 10⁴ Pa.

### Example 5: OEA/olive oil composition

A mixture of OEA and olive oil, with an OEA concentration of 4% by weight, is heated for 10 min at 70°C, cooled to 20°C and then left at this temperature for 1 hr. The resulting composition is an opalescent yellow/green gel. This gel is stable for at least 3 months at 20°C and does not show any syneresis. The resulting composition has an elastic modulus of 10⁴ Pa.

### Example 6: OEA/argan oil composition

A mixture of OEA and argan oil, at an OEA concentration of 4% by weight, is heated for 10 min at 70°C, cooled to 20°C and left at this temperature for 1 h. The resulting composition is an opalescent yellow gel. This gel is stable for at least 3 months at 20°C and does not show any syneresis. The resulting composition has an elastic modulus of 1.5.10⁴ Pa.

### Example 7: OEA/paraffin oil composition

A mixture of OEA and paraffin oil, with an OEA concentration of 4% by weight, is heated for 10 min at 70°C, then cooled to 20°C and left at this temperature for 1 h. The resulting composition is an opalescent white gel. This gel is stable for at least 3 months at 20°C and does not show any syneresis. The resulting composition has an elastic modulus of 2.5.10³ Pa.

Compositions according to Example 2 have been produced, in the same way as for Examples 3 to 7, for different OEA concentrations and/or for different fatty substances. They are presented in the following tables 1 to 5.

**Table 1: Concentration of 1% OEA (wt%) relative to the total mass:**

| Oil | Stability of the gel |
|---|---|
| Sunflower | +++ (at 5°C) |
| Argan | +++ (at 5°C) |

| | |
|---|---|
| +++: >75 days; ++: >50 days; +: >25 days. | |

**Table 2: Concentration of 2% OEA (wt%) relative to the total mass:**

| Oil | Stability of the gel |
|---|---|
| Argan | +++ |
| Paraffin | ++ |
| Duck fat | +++ (at 35°C) |

| | |
|---|---|
| +++: >75 days; ++: >50 days; +: >25 days. | |

**Table 3: Concentration of 4% OEA (wt%) relative to the total mass:**

| Oil | Stability of the gel |
|---|---|
| Sunflower (Example 3) | +++ |
| Olive (Example 5) | +++ |
| Argan (Example 6) | +++ |
| Jojoba | + |
| Paraffin (Example 7) | +++ |
| Duck fat | +++ (at 38°C) |

| | |
|---|---|
| +++: >75 days; ++: >50 days; +: >25 days. | |

**Table 4: Concentration of 6% OEA (wt%) relative to the total mass:**

| Oil | Stability of the gel |
|---|---|
| Rapeseed (Example 4) | +++ |
| Coconut | + (at 32°C) |

| | |
|---|---|
| +++: >75 days; ++: >50 days; +: >25 days. | |

**Table 5: Concentration of 10% OEA (wt%) relative to the total mass:**

| Oil | Stability of the gel |
|---|---|
| Rapeseed | + |
| Sunflower | + |

| | |
|---|---|
| +++: >75 days; ++: >50 days; +: >25 days. | |

### Conclusion

The texturizing complexes according to the invention are stable and represent therefore an alternative to known texturizing agents. Moreover, the texturizing complexes according to the invention comprise oils that are biosourced, harmless, and environment friendly.

### List of references

(1) Brouwer, I. A.; Wanders, A. J.; Katan, M. B. Effect of Animal and Industrial Trans Fatty Acids on HDL and LDL Cholesterol Levels in Humans - A Quantitative Review. PLOS ONE 2010, 5 (3), e9434. https://doi.org/10.1371/journal.pone.0009434.
(2) Zock, P. L.; Katan, M. B. Trans Fatty Acids, Lipoproteins, and Coronary Risk. Can. J. Physiol. Pharmacol. 2011, 75, 211-216. https://doi.org/10.1139/y97-012.
(3) Brouwer, I. A.; Wanders, A. J.; Katan, M. B. Trans Fatty Acids and Cardiovascular Health: Research Completed? Eur. J. Clin. Nutr. 2013, 67 (5), 541-547. https://doi.org/10.1038/ejcn.2013.43.
(4) Zhu, Y.; Bo, Y.; Liu, Y. Dietary Total Fat, Fatty Acids Intake, and Risk of Cardiovascular Disease: A Dose-Response Meta-Analysis of Cohort Studies. Lipids Health Dis. 2019, 18 (1), 91. https://doi.org/10.1186/s12944-019-1035-2.
(5) Scientific Opinion on Dietary Reference Values for Fats, Including Saturated Fatty Acids, Polyunsaturated Fatty Acids, Monounsaturated Fatty Acids, Trans Fatty Acids, and Cholesterol. EFSA J. 2010, 8 (3), 1461. https://doi.org/10.2903/j.efsa.2010.1461.
(6) Liu, A. G.; Ford, N. A.; Hu, F. B.; Zelman, K. M.; Mozaffarian, D.; Kris-Etherton, P. M. A Healthy Approach to Dietary Fats: Understanding the Science and Taking Action to Reduce Consumer Confusion. Nutr. J. 2017, 16 (1), 53. https://doi.org/10.1186/s12937-017-0271-4.
(7) Marangoni, A. G.; Co, E. D. Organogels: An Alternative Edible Oil-Structuring Method. J. Am. Oil Chem. Soc. 2012, 89 (5), 749-780. https://doi.org/10.1007/s11746-012-2049-3.
(8) Wesdorp, L. H.; Melnikov, S. M.; Gaudier, E. A. Trans Fats Replacement Solutions in Europe. In Trans Fats Replacement Solutions; Kodali, D. R., Ed.; AOCS Press, 2014; pp 287-312. https://doi.org/10.1016/B978-0-9830791-5-6.50018-6.
(9) Zetzl, A. K.; Marangoni, A. G. 10 - Structured Emulsions and Edible Oleogels as Solutions to Trans Fat. In Trans Fats Replacement Solutions; Kodali, D. R., Ed.; AOCS Press, 2014; pp 215-243. https://doi.org/10.1016/B978-0-9830791-5-6.50015-0.
(10) Davidovich-Pinhas, M.; Barbut, S.; Marangoni, A. g. Development, Characterization, and Utilization of Food-Grade Polymer Oleogels. Annu. Rev. Food Sci. Technol. 2016, 7 (1), 65-91. https://doi.org/10.1146/annurev-food-041715-033225.
(11) Scharfe, M.; Flöter, E. Oleogelation: From Scientific Feasibility to Applicability in Food Products. Eur. J. Lipid Sci. Technol. 2020, 2000213. https://doi.org/10.1002/ejlt.202000213.
(12) Fu, J.; Gaetani, S.; Oveisi, F.; Lo Verme, J.; Serrano, A.; Rodriguez de Fonseca, F.; Rosengarth, A.; Luecke, H.; Di Giacomo, B.; Tarzia, G.; Piomelli, D. Oleylethanolamide Regulates Feeding and Body Weight through Activation of the Nuclear Receptor PPAR-α. Nature 2003, 425 (6953), 90-93. https://doi.org/10.1038/nature0921.
(13) Gaetani, S.; Oveisi, F.; Piomelli, D. Modulation of Meal Pattern in the Rat by the Anorexic Lipid Mediator Oleoylethanolamide. Neuropsychopharmacology 2003, 28 (7), 1311-1316. https://doi.org/10.1038/sj.npp.1300166.
(14) Nielsen, M. J.; Petersen, G.; Astrup, A.; Hansen, H. S. Food Intake Is Inhibited by Oral Oleoylethanolamide. J. Lipid Res. 2004, 45 (6), 1027-1029. https://doi.org/10.1194/jlr.C300008-JLR200.
(15) Tutunchi, H.; Ostadrahimi, A.; Saghafi-Asl, M.; Hosseinzadeh-Attar, M.-J.; Shaken, A.; Asghari-Jafarabadi, M.; Roshanravan, N.; Farrin, N.; Naemi, M.; Hasankhani, M. Oleoylethanolamide Supplementation in Obese Patients Newly Diagnosed with Non-Alcoholic Fatty Liver Disease: Effects on Metabolic Parameters, Anthropometric Indices, and Expression of PPAR-α, UCP1, and UCP2 Genes. Pharmacol. Res. 2020, 156, 104770. https://doi.org/10.1016/j.phrs.2020.104770.
(16) Sabahi, M.; Ahmadi, S. A.; Kazemi, A.; Mehrpooya, M.; Khazaei, M.; Ranjbar, A.; Mowla, A. The Effect of Oleoylethanolamide (OEA) Add-On Treatment on Inflammatory, Oxidative Stress, Lipid, and Biochemical Parameters in the Acute Ischemic Stroke Patients: Randomized Double-Blind Placebo-Controlled Study. Oxid. Med. Cell. Longev. 2022, 2022, e5721167. https://doi.org/10.1155/2022/5721167.
(17) Davidovich-Pinhas, M.; Barbut, S.; Marangoni, A. G. The Gelation of Oil Using Ethyl Cellulose. Carbohydr. Polym. 2015, 117, 869-878. https://doi.org/10.1016/j.carbpol.2014.10.035.
(18) Ritter, H.; Sande, R. L. van de; Muller, V. Liquid Fatty Component Containing Composition. US6846507B1, January 25, 2005. https://patents.google.com/patent/US6846507B1/en (accessed 2023-02-01).
(19) Ojijo, N. K.; Neeman, I.; Eger, S.; Shimoni, E. Effects of Monoglyceride Content, Cooling Rate and Shear on the Rheological Properties of Olive Oil/Monoglyceride Gel Networks. J. Sci. Food Agric. 2004, 84 (12), 1585-1593. https://doi.org/10.1002/jsfa. 1831.
(20) Toro-Vazquez, J. F.; Morales-Rueda, J. A.; Dibildox-Alvarado, E.; Charó-Alonso, M.; Alonzo-Macias, M.; González-Chávez, M. M. Thermal and Textural Properties of Organogels Developed by Candelilla Wax in Safflower Oil. J. Am. Oil Chem. Soc. 2007, 84 (11), 989-1000. https://doi.org/10.1007/s11746-007-1139-0.
(21) Gandolfo, F. G.; Bot, A.; Flöter, E. Structuring of Edible Oils by Long-Chain FA, Fatty Alcohols, and Their Mixtures. J. Am. Oil Chem. Soc. 2004, 81 (1), 1-6. https://doi.org/10.1007/s11746-004-0851-5.
(22) Elliger, C. A.; Guadagni, D. G.; Dunlap, C. E. Thickening Action of Hydroxystearates in Peanut Butter. J. Am. Oil Chem. Soc. 1972, 49 (9), 536-537. https://doi.org/10.1007/BF02628900.

## Claims

1. A texturizing complex comprising oleoylethanolamide and at least one fatty substance.

2. The texturizing complex according to claim 1, wherein the mass concentration of the at least one fatty substance relative to the total mass of the texturizing complex is in the range from 70 to 99.9 %.

3. The texturizing complex according to any of the preceding claims, wherein the mass concentration of oleoylethanolamide relative to the total mass of the texturizing complex is in the range from 0.1 to 30 %.

4. The texturizing complex according to any of the preceding claims, wherein the fatty substance is selected from oil, butter, fat, emulsion and mixtures thereof.

5. The texturizing complex according to claim 4, wherein the oil is selected from vegetable oil, animal oil, mineral oil, synthetic oil and mixtures thereof.

6. The texturizing complex according to claim 4, wherein the butter is selected from clarified butter, ghee, shea butter, cocoa butter, peanut butter, avocado butter, nut butter and mixtures thereof.

7. The texturizing complex according to claim 4, wherein the emulsion is selected from water in oil emulsion such as margarine.

8. The texturizing complex according to claim 4, wherein the fat is selected from duck fat, ox tallow, sheep tallow, lard and mixtures thereof.

9. The texturizing complex according to any of the preceding claims, wherein the texturizing complex further comprises at least one wax.

10. The texturizing complex according to claim 9, wherein the mass concentration of the at least one wax relative to the total mass of the texturizing complex is in the range from 0.01 to 20 %.

11. A food composition comprising a texturizing complex according to any one of claims 1-10.

12. A cosmetic composition comprising a texturizing complex according to any one of claims 1-10.

13. A pharmaceutical composition comprising a texturizing complex according to any one of claims 1-10 and an active agent.

14. The pharmaceutical composition according to claim 13 for use as a medicament.

15. A process to manufacture a texturizing complex according to claim 1 wherein the process comprises:
a) a step of bringing into contact oleoylethanolamide and a fatty substance;
b) a step of gradually increasing the temperature of the mixture obtained in step a) until the dissolution of the oleoylethanolamide;
c) a step of homogenisation the mixture obtained in step b) to obtain a homogeneous mixture;
d) a step of gradually decreasing the temperature of the homogeneous mixture until the texturizing complex according to claim 1 is formed.
